# EUROPEAN PATENT APPLICATION

(11) **EP 1 460 053 A1**
(43) Date of publication of application: **22.09.2004**
(21) Application number: 02793405.8
(22) Date of filing: 26.12.2002
(51) Int. Cl.: C07C 45/35, C07C 47/22, C07C 51/25, C07C 57/05

(54) **PROCESS FOR VAPOR-PHASE CATALYTIC OXIDATION AND PROCESS FOR PRODUCTION OF (METH)ACROLEIN OR (METH)ACRYLIC ACID**

(30) Priority: 27.12.2001 JP 2001396345; 07.01.2002 JP 2002000325
(71) Applicant: Mitsubishi Chemical Corporation, Tokyo 108-0014 (JP)
(72) Inventor: YADA, Shuhei, c/o Mitsubishi Chemical Corporation, Yokkaichi-shi, Mie 510-8530 (JP); OGAWA, Yasushi, Mitsubishi Chemical Corporation, Yokkaichi-shi, Mie 510-8530 (JP); SUZUKI, Yoshiro, Mitsubishi Chemical Corporation, Yokkaichi-shi, Mie 510-8530 (JP); HOSAKA, Hirochika, Mitsubishi Chemical Corp., Yokkaichi-shi, Mie 510-8530 (JP); JINNO, Kimikatsu, Mitsubishi Chemical Corporation, Yokkaichi-shi, Mie 510-8530 (JP); SAITO, Teruo, c/o Mitsubishi Chemical Corporation, Yokkaichi-shi, Mie 510-8530 (JP)
(74) Representative: McCluskie, Gail Wilson
(86) International application number: PCT/JP2002/013608
(87) International publication number: WO 2003/055835

(57) **Abstract**

(1) In a gas-phase catalytic oxidation process for conducting gas-phase catalytic oxidation reaction using a fixed bed multipipe type reactor having reaction tubes filled with a catalyst while feeding a reaction raw gas thereinto, the catalyst is filled in each of the reaction tubes of the fixed bed multipipe type reactor to form two or more catalyst layers having different catalytic activities from each other in a direction of the oxidation reaction, and the catalyst layer disposed nearest to a reaction raw gas inlet of the reaction tube has a higher catalytic activity than that of the next catalyst layer adjacent thereto, or (2) in a process for producing (meth)acrolein or (meth)acrylic acid by subjecting a raw material of the (meth)acrolein or (meth)acrylic acid, and molecular oxygen or a molecular oxygen-containing gas to gas-phase catalytic oxidation reaction using a fixed bed multipipe type reactor having two or more catalyst layers in an axial direction of each of reaction tubes provided in the reactor, a difference between maximum and minimum reaction peak temperatures of the respective catalyst layers in the axial direction of the reaction tube is not more than 20°C. According to these processes, formation of hot spots in the catalyst can be efficiently prevented.

## Description

### TECHNICAL FIELD

The present invention relates to a gas-phase catalytic oxidation process and a process for producing (meth)acrolein or (meth)acrylic acid, and more particularly, to a gas-phase catalytic oxidation process for efficiently producing (meth)acrolein or (meth)acrylic acid by subjecting propane, propylene or isobutylene to gas-phase catalytic oxidation reaction with molecular oxygen, which process is capable of preventing formation of hot spots in a catalyst layer disposed within respective reaction tubes of a fixed bed multipipe type reactor, and enhancing a life of the catalyst used therein.

### BACKGROUND ARTS

The conventional processes for producing acrylic acid by subjecting propane, propylene or isobutylene to gas-phase catalytic oxidation reaction with molecular oxygen using a fixed bed multipipe type reactor, have been conducted using catalysts. However, these processes have such a problem that the catalyst layer filled in the respective reaction tubes of the fixed bed multipipe type reactor suffers from formation of high-temperature sites (hot spots).

Hitherto, in order to prevent the formation of hot spots in the catalyst layer, there have been proposed, for example, many methods of preventing formation of hot spots by preparing catalysts having different catalytic activities from each other, and disposing the catalyst having a lower catalytic activity at an inlet portion of the reaction tube where the concentration of the raw material is high, and the catalyst having a higher catalytic activity at a reaction gas outlet portion of the reaction tube where the concentration of the raw material is low, so as to allow the catalyst layer as a whole to exhibit the catalytic activity to the reaction.

In Japanese Patent Application Laid-open (KOKAI) No. 51-127013, there has been proposed the process for producing propylene or isobutylene in the presence of an oxidation catalyst using a fixed bed multipipe type reactor in which a supported-type catalyst and a molded catalyst that are substantially identical in composition to each other, are used in combination.

In Japanese Patent Application Laid-open (KOKAI) No. 3-294239, there has been proposed the process for producing acrolein and acrylic acid by subjecting propylene to gas-phase catalytic oxidation reaction using a fixed bed multipipe type reactor in which a plurality of catalysts exhibiting different catalytic activities from each other by varying kinds and/or amounts of alkali earth metal elements contained as catalytically active components therein, are filled in each reaction tube such that the catalytic activities thereof are increased from a raw gas inlet portion of the reaction tube toward an outlet portion thereof.

In Japanese Patent Application Laid-open (KOKAI) No. 7-10802, there has been proposed the process for producing acrylic acid by subjecting acrolein to gas-phase catalytic oxidation reaction using a fixed bed multipipe type reactor filled with a catalyst obtained by supporting a catalytically active substance containing at least molybdenum and vanadium on an inert carrier, in which the catalyst is filled in each reaction tube such that a percentage of the catalytically active substance supported thereon is sequentially increased from a raw gas inlet side of the reaction tube toward an outlet side thereof.

In Japanese Patent Application Laid-open (KOKAI) No. 9-241209, there has been proposed the process for producing acrylic acid by subjecting acrolein or an acrolein-containing gas to gas-phase catalytic oxidation reaction using a fixed bed multipipe type reactor, in which a plurality of reaction zones are provided in each reaction tube by dividing an inside thereof into two or more layers in an axial direction of the reaction tube, and the reaction zones are respectively filled with plural kinds of catalysts having different volumes from each other such that the volumes of the catalysts filled in the respective reaction zones are sequentially decreased from a raw gas inlet side of the reaction tube toward an outlet side thereof.

In Japanese Patent Application Laid-open (KOKAI) No. 8-3093, there has been proposed the process for producing acrolein and acrylic acid by subjecting propylene to gas-phase catalytic oxidation reaction with molecular oxygen using a fixed bed multipipe type reactor, in which respective reaction tubes are divided into a plurality of layers, and different kinds of catalysts are sequentially filled in each reaction tube such that the catalyst produced at a higher calcining temperature is disposed nearer to a raw gas inlet side thereof.

However, in the above conventional processes in which the catalytic activity of the respective catalysts is controlled by varying the kinds and/or amounts of the catalyst components, varying the amounts of catalytically active components supported on carrier, and adjusting the calcining temperature upon preparation of the catalysts or the volume of the respective catalysts, it is required to produce several kinds of catalysts having different catalytic activities from each other. As a result, there tend to arise problems including not only deteriorated productivity upon production of the catalysts but also difficulty in controlling the activity of these catalysts as well as non-uniformity in catalytic activity of the obtained catalysts, thereby failing to fully and satisfactorily prevent formation of hot pots irrespective of large efforts.

The present invention has been attained for solving the above problems. An object of the present invention is to provide a gas-phase catalytic oxidation process using a catalyst filled in respective reaction tubes of a fixed bed multipipe type reactor, which process is prevented from formation of hot spots in a catalyst layer, and is capable of enhancing a life of the catalyst and allowing the gas-phase catalytic oxidation reaction to be performed at a high efficiency, and further a process for efficiently producing (meth)acrolein or (meth)acrylic acid by the gas-phase catalytic oxidation process.

Another object of the present invention is to provide a process for producing (meth)acrolein or (meth)acrylic acid by subjecting a raw material of the (meth)acrolein or (meth)acrylic acid, and molecular oxygen or a molecular oxygen-containing gas to gas-phase catalytic oxidation reaction using a fixed bed multipipe type reactor having two or more catalyst layers disposed in an axial direction of each reaction tube thereof, in which temperatures of the respective catalyst layers are optimized to effectively prevent formation of hot spots therein.

### DISCLOSURE OF THE INVENTION

The oxidation reaction of olefins into unsaturated aldehydes or unsaturated acids is an exothermic reaction. Therefore, in order to prevent formation of hot spots that tend to cause undesirable side reactions and adversely affect a life of a catalyst used therein, it is important to efficiently remove heat generated during the reaction, or scatter or diffuse the heat of reaction for preventing local heat reserve.

Further, the raw olefins such as propylene are mixed with molecular oxygen (air) or steam and further, if required, with inert gas, and then fed to respective reaction tubes of a fixed bed multipipe type reactor. In this case, since the temperature of the reaction raw gas is usually lower than the reaction temperature, a preheating layer filled with an inert substance is provided at a reaction raw gas inlet portion thereof in order to increase a temperature of the raw gas to the reaction temperature.

As a result of the present inventors' earnest studies, it has been found that when the preheating layer provided at the reaction raw gas inlet portion is filled with not the inert substance but a catalyst, the temperature of the reaction raw gas can be more rapidly increased, and further formation of hot spots in the catalyst can be effectively prevented. The present invention has been attained on the basis of the above finding.

The present inventions includes a series of related aspects, and subject matters of the aspects of the present invention are as follows:
1. A gas-phase catalytic oxidation process for conducting gas-phase catalytic oxidation reaction using a fixed bed multipipe type reactor having reaction tubes each filled with a catalyst while feeding a reaction raw gas thereinto,
   the catalyst being filled in each of the reaction tubes of the fixed bed multipipe type reactor to form two or more catalyst layers having different catalytic activities from each other in a direction of the oxidation reaction, and
   the catalyst layer disposed nearest to a reaction raw gas inlet of the reaction tube having a higher catalytic activity than that of the next catalyst layer adjacent thereto.
2. A gas-phase catalytic oxidation process according to the above aspect 1, wherein the catalyst forms three or more catalyst layers, and the catalyst layers other than the catalyst layer disposed nearest to the reaction raw gas inlet are disposed such that the catalytic activities thereof are increased from the reaction raw gas inlet side of the reaction tube toward an outlet side thereof.
3. A gas-phase catalytic oxidation process according to the above aspect 1, wherein the catalytic layer disposed nearest to the reaction raw gas inlet of the reaction tube is filled therein such that a temperature of the reaction raw gas introduced thereinto is higher than at least a temperature of a heating medium flowing outside the reaction tube.
4. A gas-phase catalytic oxidation process according to the above aspect 1, wherein a difference between maximum and minimum reaction peak temperatures of the respective catalyst layers in an axial direction of the reaction tube is not more than 20°C.
5. A gas-phase catalytic oxidation process according to the above aspect 1, wherein at least one catalyst layer is controlled in catalytic activity thereof by mixing an inert substance therein.
6. A process for producing (meth)acrolein or (meth)acrylic acid using the gas-phase catalytic oxidation process as defined in any of the above aspects 1 to 5 to produce (meth)acrolein or (meth)acrylic acid by oxidizing propane, propylene or isobutylene with molecular oxygen, or to produce (meth)acrylic acid by oxidizing (meth)acrolein.
7. A process for producing (meth)acrolein or (meth)acrylic acid by subjecting a raw material of the (meth)acrolein or (meth)acrylic acid, and molecular oxygen or a molecular oxygen-containing gas to gas-phase catalytic oxidation reaction using a fixed bed multipipe type reactor having two or more catalyst layers disposed in an axial direction of each of reaction tubes provided in the reactor, wherein a difference between maximum and minimum reaction peak temperatures of the respective catalyst layers in the axial direction of the reaction tube is not more than 20°C.
8. A process according to the above aspect 7, wherein a difference between maximum and minimum reaction peak temperatures of a plurality of the catalyst layers is not more than 10°C.
9. A process according to the above aspect 7, wherein at least one catalyst layer is controlled in catalytic activity thereof by mixing an inert substance therein.
10. A process according to the above aspect 7, wherein the number of the catalyst layers is 2 to 5.

The present invention is described in detail below.

First, the gas-phase catalytic oxidation process of the present invention is explained.

The gas-phase catalytic oxidation reaction using the fixed bed multipipe type reactor has been extensively used to produce (meth)acrolein or (meth)acrylic acid by reacting propylene or isobutylene with molecular oxygen or a molecular oxygen-containing gas in the presence of a composite oxide catalyst, and this reaction includes the step of producing (meth)acrolein from propylene or isobutylene, and further the step of producing (meth)acrylic acid from the (meth)acrolein.

In general, the gas-phase catalytic oxidation reaction includes a front stage reaction for producing acrylic acid by oxidizing propane in the presence of a Mo-V-Te-based composite oxide catalyst, a Mo-V-Sb-based composite oxide catalyst, etc., or producing mainly (meth)acrolein by oxidizing propylene or isobutylene in the presence of a Mo-Bi-based composite oxide catalyst, and a rear stage reaction for producing (meth)acrylic acid by oxidizing the (meth)acrolein obtained in the front stage reaction in the presence of a Mo-V-based composite oxide catalyst.

As typical methods of industrialized gas-phase catalytic oxidation, there are known one-pass method, unreacted propylene recycling method and combustion exhaust gas recycling method.

The one-pass method is such as method including a front stage reaction in which propylene, air and steam are mixed with each other and fed through a reaction raw gas inlet to convert the mixed raw gas into mainly acrolein and acrylic acid, and a rear stage reaction in which the resultant outlet gas from the front stage reaction which includes the above reaction products without separation thereof, is fed to the fixed bed multipipe type reactor to oxidize acrolein contained in the outlet gas into acrylic acid. At this time, there may also be generally used the method of adding air and steam required for the rear stage reaction to the outlet gas from the front stage reaction, and feeding the resultant mixed gas to the rear stage reaction.

The unreacted propylene recycling method is such a method in which an acrylic acid-containing reaction product gas obtained at an outlet of a rear stage reaction is introduced into an acrylic acid-collecting apparatus to recover the acrylic acid in the form of an aqueous solution, and a part of an exhaust gas containing unreacted propylene is fed from the collecting apparatus to a reaction raw gas inlet for a front stage reaction to recycle a part of the unreacted propylene.

The combustion exhaust gas recycling method is such a method in which an acrylic acid-containing reaction product gas obtained at an outlet of a rear stage reaction is introduced into an acrylic acid-collecting apparatus to recover the acrylic acid in the form of an aqueous solution, a whole amount of the exhaust gas from the collecting apparatus is catalytically combustion-oxidized to convert unreacted propylene or the like contained in the exhaust gas into mainly carbon dioxide and water, and adding a part of the obtained combustion exhaust gas to the reaction raw gas fed to an inlet for the front stage reaction.

In the gas-phase catalytic oxidation process of the present invention, the reaction raw gas may be fed to the reaction raw gas inlet by the above respective methods. However, the reaction raw gas used in the present invention is not limited to those fed by the above methods as long as the raw gas is usable in the gas-phase catalytic oxidation reaction for producing acrylic acid.

The catalyst used in the gas-phase catalytic oxidation process of the present invention is preferably such a catalyst for production of acrylic acid which is filled in respective reaction tubes of the fixed bed multipipe type reactor used for producing (meth)acrolein or (meth)acrylic acid. Specific examples of the catalyst may include the following catalysts.

The catalyst used in the gas-phase catalytic oxidation reaction for producing acrylic acid, may include those used in the front stage reaction for converting olefins into unsaturated aldehydes or unsaturated acids, and those used in the rear stage reaction for converting the unsaturated aldehydes into the unsaturated acids. The process of the present invention can be applied to both the reactions.

As the catalyst used in the front stage reaction, there may be exemplified those catalysts represented by the following general formula (I):

MoₐW_{b}Bi_{c}Fe_{d}AₑB_{f}C_{g}DₕEᵢOₓ (I)

wherein Mo is molybdenum; W is tungsten; Bi is bismuth; Fe is iron; A is at least one element selected from the group consisting of nickel and cobalt; B is at least one element selected from the group consisting of sodium, potassium, rubidium, cesium and thallium; C is at least one element selected from the group consisting of alkali earth metals; D is at least one element selected from the group consisting of phosphorus, tellurium, antimony, tin, cerium, lead, niobium, manganese, arsenic, boron and zinc; E is at least one element selected from the group consisting of silicon, aluminum, titanium and zirconium; O is oxygen; and a, b, c, d, e, f, g, h, i and x are atomic ratios of Mo, W, Bi, Fe, A, B, C, D, E and O, respectively, with the proviso that when a is 12 (a = 12), 0 ≤ b ≤ 10, 0 < c ≤ 10 (preferably 0.1 ≤ c ≤ 10), 0 < d ≤ 10 (preferably 0.1 ≤ d ≤ 10), 2 ≤ e ≤ 15, 0 < f ≤ 10 (preferably 0.001 ≤ f ≤ 10), 0 ≤ g ≤ 10, 0 ≤ h ≤ 4, 0 ≤ i ≤ 30, and x is a value determined by oxidation degrees of the respective elements.

As the catalyst used in the rear stage reaction of the present invention, there may be exemplified those catalysts represented by the following general formula (II):

MoₐV_{b}W_{c}Cu_{d}XₑY_{f}O_{g} (II)

wherein Mo is molybdenum; V is vanadium; W is tungsten; Cu is copper; X is at least one element selected from the group consisting of Mg, Ca, Sr and Ba; Y is at least one element selected from the group consisting of Ti, Zr, Ce, Cr, Mn, Fe, Co, Ni, Zn, Nb, Sn, Sb, Pb and Bi; O is oxygen; and a, b, c, d, e, f and g are atomic ratios of Mo, V, W, Cu, X, Y and O, respectively, with the proviso that when a is 12 (a = 12), 2 ≤ b ≤ 14, 0 ≤ c ≤ 12, 0 < d ≤ 6, 0 ≤ e ≤ 3, 0 ≤ f ≤ 3, and g is a value determined by oxidation degrees of the respective elements.

The above catalysts may be produced, for example, by the method described in Japanese Patent Application Laid-open (KOKAI) No. 63-54942.

The catalyst used in the present invention may be in the form of a molded catalyst produced by an extrusion-molding method or a tablet-forming method, or may be in the form of a supported catalyst obtained by supporting a composite oxide as a catalyst component on an inert carrier such as silicon carbide, alumina, zirconium oxide and titanium oxide.

The shape of the catalyst used in the present invention is not particularly restricted, and the catalyst may be of any shape such as a spherical shape, a cylindrical shape, a hollow cylindrical shape, a ring shape, a star-like shape and an amorphous shape. Of these shapes, the ring shape is more preferred since the use of such a ring-shaped catalyst has an effect of preventing heat reserve at hot spot portions.

The activity of the catalyst used in the present invention is controlled by conventional methods such as, for example, a method of diluting the above catalyst with an inert substance, a method of adjusting an amount of the catalyst supported on an inert carrier, a method of adjusting catalyst properties such as volume, pore volume and pore distribution, and a method of adjusting production conditions of the catalyst such as a calcining temperature. Namely, the activity of the catalyst is in inverse proportion to the amount of the inert substance used. As the inert substance used in the present invention, there may be used any substances that are kept stable under the reaction conditions for production of acrylic acid and have no reactivity to raw materials such as olefins as well as reaction products such as unsaturated aldehydes and unsaturated acids. Specific examples of the inert substance may include alumina, silicon carbide, silica, zirconium oxide, titanium oxide or the like, i.e., those usable as a carrier for catalysts. The shape of the inert substance is not particularly restricted similarly to that of the catalyst, and the inert substance may be of any shape such as a spherical shape, a cylindrical shape, a ring shape and an amorphous shape. The size of the inert substance may be determined in view of diameter and pressure loss of the reaction tube, etc. As described above, the activity of the catalyst may be controlled by diluting the catalyst with the inert substance, thereby forming catalyst layers having different catalytic activities from each other.

The fixed bed multipipe type reactor used in the present invention is not particularly restricted, and may be those reactors generally used in industrial fields.

In the present invention, when the catalyst is filled in the respective reaction tubes of the fixed bed multipipe type reactor, two or more catalyst layers having different catalytic activities from each other are formed therein in a direction of the oxidation reaction such that the catalyst layer disposed nearest to a reaction raw gas inlet of the reactor tube (hereinafter referred to merely as "first layer") has a higher catalytic activity than that of the next catalyst layer adjacent thereto (hereinafter referred to merely as "second layer"). When the first layer is filled with the catalyst having a higher catalytic activity than that of the second layer, even the raw gas having a low temperature can be reacted therein.

Accordingly, the length of the first layer disposed in the respective reaction tubes of the fixed bed multipipe type reactor as well as the activity of the catalyst filled therein are preferably controlled such that at least the temperature of the reaction raw gas introduced thereinto is not less than at least a temperature of a heating medium flowing outside the reaction tube. Although the temperature of the heating medium is different between the inlet and outlet of the respective reaction tubes of the reactor, the inlet temperature of the heating medium is usually used as a standard. The length of the first layer filled is preferably selected such that a reaction peak temperature of the first layer is substantially identical to that of the second layer. The length of the catalyst layer filled can be readily calculated from the mass balance and heat balance by determining the activity of the catalyst filled (in the case of diluted catalyst, the activity of the catalyst including a diluting material), the temperature of the reaction raw gas, and the reaction temperature and reaction conditions. A preferable combination of the length of the catalyst layer and the catalytic activity may be selected according to the reaction conditions.

The respective reaction tubes of the fixed bed multipipe type reactor are filled with three or more catalyst layers. In this case, when the catalyst layers other than the catalyst layer nearest to the reaction raw gas inlet are disposed such that the catalytic activities thereof are increased from the raw gas inlet side toward the outlet side, it becomes possible to prevent formation of hot spots therein as well as heat reserve in the hot spot portions. As a result, the reaction can be conducted safely and efficiently, thereby achieving a high productivity without damage to a life of the catalyst.

The number of the catalyst layers may be appropriately selected so as to attain maximum effects. When the number of the catalyst layers is too large, there tend to be caused additional problems such as complicated filling work. Therefore, the number of the catalyst layer filled is preferably about 3 to 5.

Also, the compositions of the respective catalyst layers used in the present invention are not particularly restricted as long as these catalyst layers are arranged such that the catalytic activity of the first layer is higher than that of the second layer, and the catalyst activities of the second and subsequent layers are increased toward the outlet side. The compositions of these catalyst layers may be identical to or different from each other. A preheating layer is preferably provided on an upstream side of the catalyst layers. The length of the preheating layer is usually 5 to 20% and preferably 10 to 20% of an entire length of the catalyst layers.

Next, the process for producing (meth)acrolein or (meth)acrylic acid according to the present invention is explained.

In the present invention, as the raw material, there may be used the following compounds. Namely, there may be used propylene for production of acrolein, and isobutylene for production of methacrolein. Meanwhile, since (meth)acrolein is an intermediate product for production of (meth)acrylic acid, the acrylic acid may be produced from raw propylene via acrolein, and methacrylic acid may be produced from raw isobutylene via methacrolein. Further, propane may also be used as a raw material for production of acrylic acid. Further, as the molecular oxygen-containing gas, there may be usually used air.

Next, the present invention is illustratively explained concerning the process for producing acrolein and acrylic acid from raw propylene. As described above, the typical examples of the industrialized processes for producing acrolein and acrylic acid from raw propylene include one-pass method, unreacted propylene recycling method and combustion exhaust gas recycling method. However, the reaction method used in the present invention is not limited to any methods including these three methods.

The Mo-Bi-based composite oxide catalyst used in the front stage reaction for producing mainly acrolein (reaction for producing unsaturated aldehydes or unsaturated acids from olefins) is composed of compounds represented by the above general formula (I). Also, the Mo-V-based composite oxide catalyst used in the rear stage reaction for producing acrylic acid by oxidizing acrolein (reaction for producing unsaturated acids from unsaturated aldehydes) is composed of compounds represented by the above general formula (II).

In the present invention, there is used the fixed bed multipipe type reactor provided with two or more catalyst layers in an axial direction of each reaction tube thereof, and further a difference between maximum and minimum reaction peak temperatures of the respective catalyst layers in the axial direction of the reaction tube is not more than 20°C. Meanwhile, the "reaction peak temperature" means a peak temperature of each catalyst layer.

When the temperature difference is more than 20°C, it may be difficult to produce acrolein and acrylic acid as aimed products at a high yield. The difference between maximum and minimum reaction peak temperatures of the respective catalyst layers in the axial direction of the reaction tube is preferably not more than 10°C.

In the present invention, the method for controlling the difference between maximum and minimum reaction peak temperatures of the respective catalyst layers in the axial direction of the reaction tube to not more than 20°C is not particularly restricted. For example, there may be used a method of appropriately varying the ratio of the inert substance to the catalyst, the shape of the catalyst, the kind of the catalyst (such as composition and calcining temperature upon production of the catalyst) or the like. Further, in the case of supported catalyst, there may also be used a method of varying the amount of catalytically active component supported on a carrier.

The number of the catalyst layers formed in an axial direction of the reaction tube of the fixed bed multipipe type reactor is not particularly restricted. However, when the number of the catalyst layers formed is too large, the catalyst filling work tends to require much labor. Therefore, the number of the catalyst layers formed is usually 2 to 5. An optimum length of the respective catalyst layers may be appropriately determined according to the kind of catalyst, the number of catalyst layers, reaction conditions, etc., so as to exhibit the largest effects of the present invention. The length of the respective catalyst layers is usually 10 to 80% and preferably 20 to 70% of an entire length of the reaction tube.

### PREFERRED EMBODIMENT OF THE PRESENT INVENTION

The present invention is described in more detail by Examples, but the Examples are only illustrative and not intended to limit the scope of the present invention.

### <Examples corresponding to gas-phase catalytic oxidation process of the present invention>

### Example 1:

A ring-shaped catalyst having the following composition (atomic ratios) as a gas-phase catalytic oxidation catalyst for propylene was prepared by the method described in Japanese Patent Application Laid-open (KOKAI) No. 63-54942.

Mo:Bi:Co:Fe:Na:B:K:Si:O = 12:1:0.6:7:0.1:0.2:0.1:18:X wherein X is a value determined by oxidation degrees of the respective metal elements.

A stainless steel reaction tube of a double tube structure having an inner diameter of 27 mm and a length of 5 m was used, and a niter was used as a heating medium to control the reaction tube at a uniform temperature.

The catalyst prepared by the above method was filled in each reaction tube to form a catalyst layer having a height of 1.5 m therein. Further, a mixture containing the above catalyst and alumina balls at a mixing ratio (volume %) of 70%:30%, a mixture containing the above catalyst and alumina balls at a mixing ratio (volume %) of 60%:40%, and a mixture containing the above catalyst and alumina balls at a mixing ratio (volume %) of 80%:20%, were successively filled in the reaction tube to form additional three catalyst layers having heights of 0.65 m, 0.65 m and 0.2 m, respectively, on the firstly filled catalyst layer in this order.

Then, a 200°C mixed gas as a reaction raw gas composed of 8 mol% of propylene, 67 mol% of air and 25 mol% of steam was fed into the respective reaction tubes of a fixed bed multipipe type reactor from a top thereof such that the reaction raw gas was contacted with the catalyst for 3.5 seconds. In addition, the temperature of the niter was controlled so as to attain a propylene conversion rate of 98%. The results are shown in Table 1.

### Reference Example 1

The catalyst prepared in Example 1 was filled in each reaction tube to form a catalyst layer having a height of 1.5 m therein. Further, a mixture containing the above catalyst and alumina balls at a mixing ratio (volume %) of 70%:30%, a mixture containing the above catalyst and alumina balls at a mixing ratio (volume %) of 60%:40%, and alumina balls solely, were successively filled in the reaction tube to form additional three layers having heights of 0.65 m, 0.65 m and 0.2 m, respectively, on the firstly filled catalyst layer in this order. Then, the reaction was conducted under the same conditions as used in Example 1. In addition, the temperature of the niter was controlled so as to attain a propylene conversion rate of 98%. The results are shown in Table 1.

### Reference Example 2

The catalyst prepared in Example 1 was filled in each reaction tube to form a catalyst layer having a height of 1.5 m therein. Further, a mixture containing the above catalyst and alumina balls at a mixing ratio (volume %) of 70%:30%, and a mixture containing the above catalyst and alumina balls at a mixing ratio (volume %) of 60%:40%, were successively filled in the reaction tube to form additional two layers having heights of 0.65 m and 0.85 m, respectively, on the firstly filled catalyst layer in this order. Then, the reaction was conducted under the same conditions as used in Example 1. In addition, the temperature of the niter was controlled so as to attain a propylene conversion rate of 98%. The results are shown in Table 1.

**Table 1**

| | Niter temperature (°C) | Hot spot temperature (°C) | Yield of acrylic acid and acrolein (%) |
|---|---|---|---|
| Example 1 | 325 | 370 | 92.5 |
| Reference Example 1 | 335 | 403 | 91.6 |
| Reference Example 2 | 332 | 395 | 91.8 |

### <Examples corresponding to process for producing

### (meth)acrolein or (meth)acrylic acid according to the present invention>

### Example 2: (Production of acrolein)

As a reactor, there was used a stainless steel reactor of a double tube structure having an inner diameter of 27 mm and a length of 5 m. A Mo-Bi-Fe-based composite oxide catalyst prepared by an ordinary method was used as a reaction catalyst, and alumina balls were used as a diluting material to be mixed with the catalyst. Three kinds of mixtures containing the catalyst and alumina balls at different mixing ratios (volume %) were prepared and filled in each reaction tube of the reactor to form three catalyst layers therein. More specifically, the first layer (on a raw gas inlet side) had a height of 1 m and was composed of 29% of the catalyst and 71% of the alumina balls; the second layer had a height of 1 m and was composed of 44% of the catalyst and 56% of the alumina balls; and the third layer (on a reaction gas outlet side) had a height of 2 m and was composed of 87% of the catalyst and 13% of the alumina balls.

A molten alkali metal nitrate (niter) as a heating medium was fed to the reactor to control the reactor at a uniform temperature. Further, a mixed raw gas composed of 7 mol% of propylene, 70 mol% of air and 23 mol% of steam was fed to the reactor such that the raw gas was contacted with the catalyst for 3.5 seconds, thereby obtaining acrolein. At that time, the temperature of the heating medium was controlled so as to attain a propylene conversion rate of 98%. The reaction conditions, reaction peak temperatures of the respective catalyst layers, and yields of acrolein and acrylic acid obtained are shown in Table 2.

### Example 3 and Reference Examples 3 and 4 (Production of acrolein)

The same procedure as defined in Example 2 was conducted except that upon formation of the reaction catalyst layers, the percentages of the catalysts used in the respective catalyst layers were varied as shown in Table 3, thereby conducting the reaction. The results are shown in Table 3. Meanwhile, the heights of the respective catalyst layers were the same as those used in Example 2.

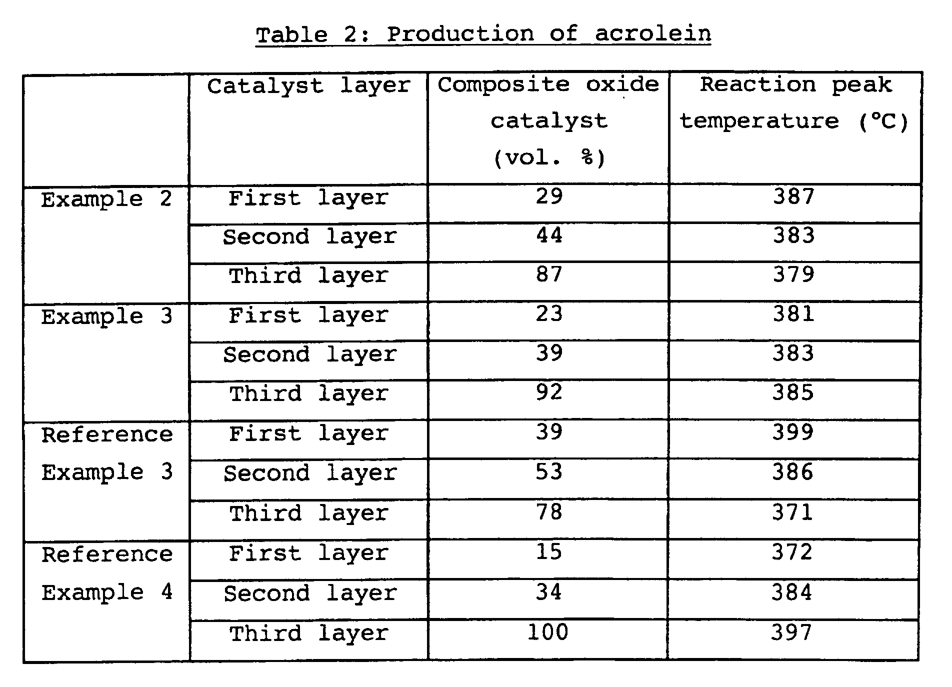

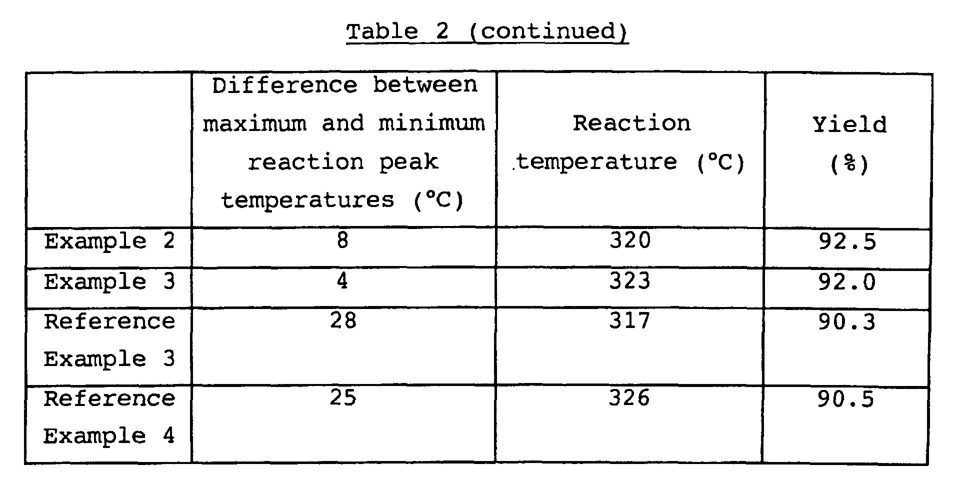

### Example 4: (Production of acrylic acid)

Two reactors of the same type as used in Example 2 were used as a front stage reactor and a rear stage reactor. The structure of the catalyst layers formed in the front stage reactor was the same as that in Example 2. The catalyst layers filled in the rear stage reactor were prepared as follows. That is, a Mo-V-Sb-based composite oxide catalyst prepared by an ordinary method was used as a reaction catalyst, and alumina balls were used as a diluting material to be mixed with the catalyst. Two kinds of mixtures containing the catalyst and alumina balls at different mixing ratios (volume %) were prepared and filled in the rear stage reactor to form two catalyst layers therein. More specifically, the first layer (on a raw gas inlet side) had a height of 1 m and was composed of 50% of the catalyst and 50% of the alumina balls; and the second layer had a height of 1.5 m and was composed of 80% of the catalyst and 20% of the alumina balls.

A molten alkali metal nitrate (niter) as a heating medium was fed to the reactor to control the reactor at a uniform temperature. Further, a mixed raw gas composed of 7 mol% of propylene, 70 mol% of air and 23 mol% of steam was fed to the front stage reactor such that the raw gas was contacted with the catalyst for 3.5 seconds, and then the resultant reaction gas was removed from the rear stage reactor, thereby obtaining acrylic acid. At that time, the temperature of the heating medium was controlled so as to attain an acrylic acid conversion rate of 99%. The reaction conditions, reaction peak temperatures of the respective catalyst layers in the rear stage reactor, and yields of acrylic acid are shown in Table 3.

### Reference Example 5 (Production of acrylic acid)

The same procedure as defined in Example 4 was conducted except that upon formation of the catalyst layers in the rear stage reactor, the percentages of the catalysts used in the respective catalyst layers were varied as shown in Table 3, thereby conducting the reaction. The results are shown in Table 3. Meanwhile, the heights of the respective catalyst layers were the same as those in Example 4.

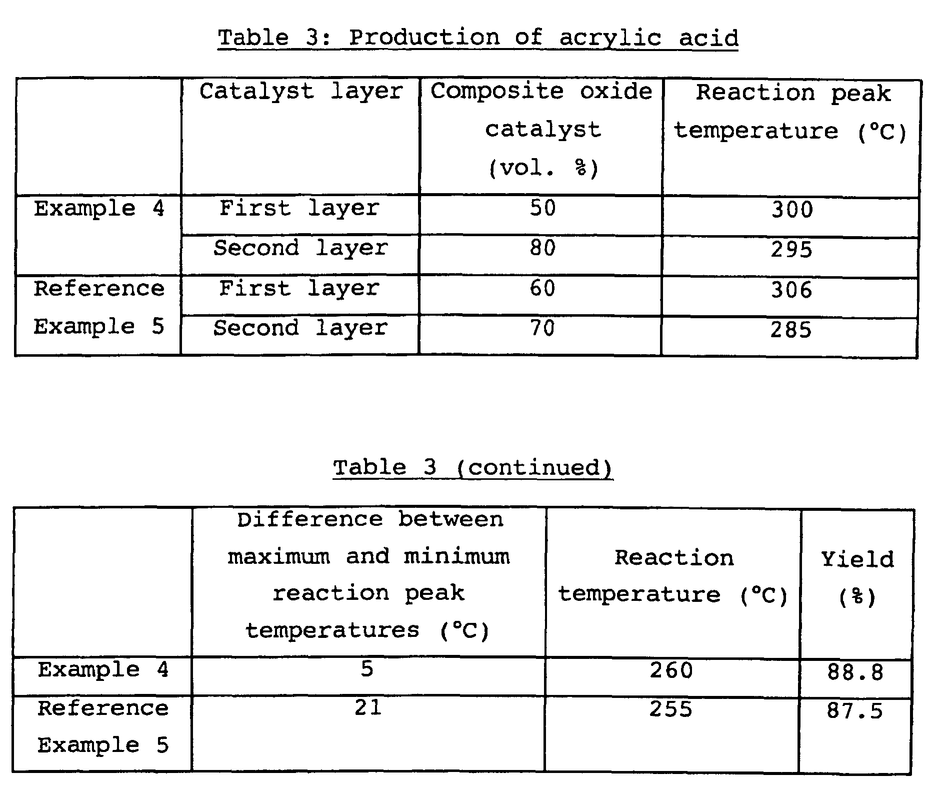

### INDUSTRIAL APPLICABILITY

According to the present invention, a plurality of catalyst layers divided in an axial direction of a reaction tube are formed such that a reaction gas inlet portion (first layer) is filled with a catalyst having a higher catalytic activity than that of the next layer (second layer), thereby efficiently preventing formation of hot spots therein. In addition, in the process of the present invention, the formation of hot spots can be effectively prevented by optimizing the temperatures of the respective catalyst layers. As a result, according to the present invention, excessive oxidation reaction can be prevented, so that (meth)acrolein and (meth)acrylic acid are produced at a high yield. Further, the catalyst can be prevented from suffering from thermal deterioration, and enhanced in life thereof, so that the reaction can be conducted at a high space velocity, resulting in a high productivity.

## Claims

1. A gas-phase catalytic oxidation process for conducting gas-phase catalytic oxidation reaction using a fixed bed multipipe type reactor having reaction tubes each filled with a catalyst while feeding a reaction raw gas thereinto,
the catalyst being filled in each of the reaction tubes of the fixed bed multipipe type reactor to form two or more catalyst layers having different catalytic activities from each other in a direction of the oxidation reaction, and
the catalyst layer disposed nearest to a reaction raw gas inlet of the reaction tube having a higher catalytic activity than that of the next catalyst layer adjacent thereto.

2. A gas-phase catalytic oxidation process according to claim 1, wherein the catalyst forms three or more catalyst layers, and the catalyst layers other than the catalyst layer disposed nearest to the reaction raw gas inlet are disposed such that the catalytic activities thereof are increased from the reaction raw gas inlet side of the reaction tube toward an outlet side thereof.

3. A gas-phase catalytic oxidation process according to claim 1, wherein the catalytic layer disposed nearest to the reaction raw gas inlet of the reaction tube is filled therein such that a temperature of the reaction raw gas introduced thereinto is higher than at least a temperature of a heating medium flowing outside the reaction tube.

4. A gas-phase catalytic oxidation process according to claim 1, wherein a difference between maximum and minimum reaction peak temperatures of the respective catalyst layers in an axial direction of the reaction tube is not more than 20°C.

5. A gas-phase catalytic oxidation process according to claim 1, wherein at least one catalyst layer is controlled in catalytic activity thereof by mixing an inert substance therein.

6. A process for producing (meth)acrolein or (meth)acrylic acid using the gas-phase catalytic oxidation process as defined in any of claims 1 to 5 to produce (meth)acrolein or (meth)acrylic acid by oxidizing propane, propylene or isobutylene with molecular oxygen, or to produce (meth)acrylic acid by oxidizing (meth)acrolein.

7. A process for producing (meth)acrolein or (meth)acrylic acid by subjecting a raw material of the (meth)acrolein or (meth)acrylic acid, and molecular oxygen or a molecular oxygen-containing gas to gas-phase catalytic oxidation reaction using a fixed bed multipipe type reactor having two or more catalyst layers disposed in an axial direction of each of reaction tubes provided in the reactor, wherein a difference between maximum and minimum reaction peak temperatures of the respective catalyst layers in the axial direction of the reaction tube is not more than 20°C.

8. A process according to claim 7, wherein a difference between maximum and minimum reaction peak temperatures of a plurality of the catalyst layers is not more than 10°C.

9. A process according to claim 7, wherein at least one catalyst layer is controlled in catalytic activity thereof by mixing an inert substance therein.

10. A process according to claim 7, wherein the number of the catalyst layers is 2 to 5.
